# EUROPEAN PATENT APPLICATION

(11) **EP 2 071 050 A1**
(43) Date of publication of application: **17.06.2009**
(21) Application number: 08425014.1
(22) Date of filing: 10.01.2008
(51) Int. Cl.: C23C 14/02, C23C 14/06, C23C 14/34, C23C 14/54

(54) **Osteointegration process for surgical prosthesis**

(30) Priority: 23.01.2007 IT RM20070033
(71) Applicant: Romana Film Sottili Srl, Carsoli (AQ) (IT); Scandurra, Roberto, 00147 Roma (IT)
(72) Inventor: Misiano, Carlo Prof., 00161 Roma (IT); Scandurra, Roberto Prof., 00147 Roma (IT)

(57) **Abstract**

The invention relates a vacuum thin films deposition process on surgical prosthesis, metallic or not, to enhance their osteointegration and to protect them from alteration induced by biological environment in which are inserted. The invention especially relates dental implants and orthopaedic prosthesis in Titanium. The superficial treatment obtainable, by the present process, gives the advantage to reduce, also more than fifty per cent, the necessary time for a full osteointegration of the prosthesis in side the bon in which said prosthesis is implanted. The invention can be placed in the field of human necessity, more precisely in the medical field regarding surgical prosthesis, especially for dental implants and orthopaedic prosthesis. The invention can be positioned in the processes under vacuum deposition field.

## Description

The invention relates a vacuum thin films deposition process on surgical prosthesis, metallic or not, to enhance their osteointegration and to protect them from alteration induced by biological environment in which are inserted. The invention especially relates dental implants and orthopaedic prosthesis in Titanium. The superficial treatment obtainable with the process in question gives the advantage to reduce, also more then fifty per cent, the necessary time for a full osteointegration of the prosthesis in side the bone in which said prosthesis was implanted. A previous technique, subject of a previous patent, presents difficulties relating its industrial use, in fact it is based on a deposition process using pulsed laser ablation, "PLAD", for very small surfaces coating, but it is not able to guarantee the necessary uniformity of thickness and characteristics in complicated samples like dental implants screws: all that results from accurate testing.

This and other drawbacks have been overcome by the present invention, that, in addition, allows the simultaneous coating of many samples (pieces) improving osteointegration characteristics and minimizing the rejection risk. The used thin film vacuum deposition technique, belongs to the reactive deposition field, in which the vaporized material, in this case Titanium, reacts during the deposition with an organic gas inserted in the process chamber. As known, the improvement of the prosthesis osteointegration characteristics, depends, beside the deposited film type, also from the surgical prosthesis surface roughness. With the proposed process, subject of the present patent requested, said surface roughness is realized by a sandblasting process, using micro spheres of Zirconium dioxide, ZrO₂, of optimal diameter around 120 microns, by obtaining in this way a satisfactory osteointegration. The deposited thin film composition is, in a rough outline, a mixture of the following compounds: Titanium carbide, Titanium dioxide, TiO₂, Titanium sub-oxides, TiOx, and, in addition, Carbon both bonded with other Carbon atoms and with Oxygen atoms. With regard to the elemental composition, that is of the main elements constituting the treatments, Carbon, Titanium, Oxygen, the typical percentages are:

Carbon between 25% and 36%, Titanium between 25% and 30%, Oxygen from 33% to 40%.

With reference to the chemical bounds of the main elements which constitute the treatment, typical percentages of the Titanium compounds are: TiC between 35% and 38%, TiO₂ between 30% and 37%, TiOx between 26% and 34%. The Carbon compounds have a majority of the bond C-C about 50% with values about 35% of the bonds with Titanium and the remaining percentage of the Oxygen bonds.

The invention will be in the following described for an indicative and not limitative scope, with reference to the single figure which will improve the understanding of the invention, taking present that the invention is essentially represented by a process performed on substrates in a "Ion Plating Pasma Assisted" plant, as specified in the following.

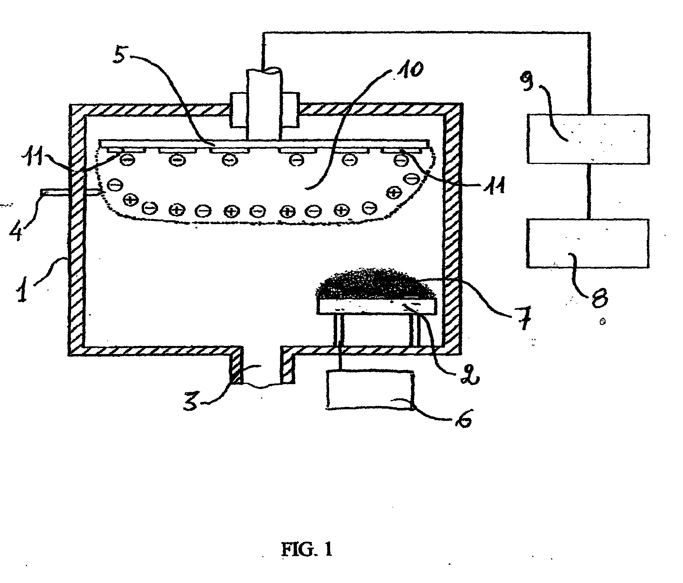

Fig. 1 - Schematic representation of a Ion Plating Plasma Assisted plant, with a "magnetron Sputtering" source. The deposition process, which is the most innovative aspect of the invention, for which a patent defence is requested, is made in a vacuum plant like a Ion Plating Plasma Assisted, with a Reactive Magnetron Sputtering source.

In the figure are visible:
- **1**: **Vacuum plant**
- 2: Magnetron sputtering source
- 3: Pumping group connections
- 4: Process gases inlet
- 5: Biased substrate holder
- 6: DC generator
- 7: DC plasma
- 8: RF generator
- 9: Matching network
- 10: RF plasma
- 11: Substrates.

The Magnetron Sputtering 2 source is generally supplied in direct voltage, while the substrate holder 5 is generally biased through a RF power supply, 13,56 MHz. The substrate holder 5 can be rotating or fixed in front of the source 2, preferably at a distance from 5 to 15 centimetres. The material on the Magnetron Sputtering source is Titanium. The process atmosphere is constituted, in some phases, by Argon, while in the reactive phase it is an Argon and Ethylene mixture. In the following the process and relative parameters are described.

The substrates 5, generally Titanium, are at first, cleaned by a soap washing, when necessary and then by a solvent like hexane. The substrates are then mounted on the substrates holder 5, with direct contact with the RF biased element 10 which generates the plasma, taking care to not pollute the surface. Then the vacuum chamber 1 is closed and the vacuum is effected until a pressure of about 10⁻⁵ Torr. Of course the pumping system is made in a way to not introduce pollution inside the chamber. A flux of Argon is introduced until the pressure reaches a value between 2 and 3x10⁻³ Torr. Then, an ionic cleaning process of the substrates is performed activating an RF discharge with a power density of about 0.5 Watt/cm², with a voltage of about 500Volts for a five minutes duration typically. Then the RF discharge is interrupted and after the substrates have been shielded, in respect of the Magnetron Sputtering source, this is supplied by a DC tension of about 400 Volts, with a power density of about 10 Watt/cm², activating a plasma discharge for the cleaning and the decontamination of the Titanium surface, for a duration of about five minutes. Then the DC voltage is interrupted and the shutter in front of the substrates is removed. Successively an adherence layer of Titanium is deposited in "Ion Plating" mode, with an RF substrates bias of about 500 Volts and a power density of about 0,25 Watt/cm², while the Magnetron Sputtering source is supplied in DC, with a power density of about 5Watt/cm², for a duration of about two minutes. During this deposition phase of the adherence coating, the Titanium deposition rate is accurately measured, by a thickness monitor, for instance using an oscillating quartz. Maintaining both the DC and RF supply, is introduced a flux of Ethylene which produces, at the same powers, a reduction of the deposition rate which mast be reduced until a value of about 90% of the deposition rate in pure Argon atmosphere. This flux of Ethylene is equivalent to a partial pressure of about 6 x 10⁻⁵ Torr. The deposition is so performed maintaining these parameters constant until to a thickness of about 1,5 microns. At this point the DC and RF supply and the flux of the process gases are interrupted, and after the vent of the plant, the substrates, so ready, are dismounted from the substrates holder.

## Claims

1. Process, for coatings for osteointegration of surgical prosthesis, to perform using an Ion Plating Plasma Assisted plant, with a "reactive Magnetron Sputtering" source, being constituted, said plant, generally, by a vacuum chamber (1), sputtering source (2), for instance Magnetron, vacuum connection (3) to the pumping group, plasma, for instance DC (7), process gases input (4), substrate holder biased (5), bias generator, for instance DC (6), RF generator (8), matching network (9), RF plasma (10), **characterised**, said process to be realized by the following steps :
- the substrates (11), usually Titanium, are previously cleaned by a soap washing, if necessary, and then by a solvent like Hexane;
- substrates (11) are mounted on the biased substrate holder (5) in direct contact with the RF biased element (10), taking care do not pollute the surface;
- the vacuum chamber (1) is closed and pumped until a pressure lower then 10⁻⁵ Torr;
- a flux of Argon is introduced until to reach a pressure value between 2 and 3 x 10⁻³ Torr;
- a cleaning of substrates (11) is performed starting an RF discharge with a power density of about 0.5Watt/cm² with a voltage of about 500 Volts, for a duration of about five minutes;
- the RF discharge is interrupted and after shielding the substrates (11), in respect of the Magnetron sputtering source (2), this, (the Magnetron sputtering source) is supplied by a DC voltage of 400 Volts tension about, with a power density of about 10 Watt/cm², activating a plasma discharge for the cleaning and the decontamination of the Titanium surface, for a duration for about five minutes;
- the supply DC is interrupted and the shutter in front of the substrates (11) is removed;
- successively, an adherence layer of Titanium is deposited, in Ion Plating mode, with an RF bias of the substrates (11) of about 500 Volts and with a power density of 0,25 Watt/cm², while the Magnetron Sputtering source is supplied in DC with a power density of about 5Watt/cm², for a duration of about two minutes; during this deposition phase of the adherence layer, the Titanium deposition rate is accurately measured by, for instance, an oscillating quartz thickness monitor;
- maintaining both the DC and RF supply, a flux of Ethylene is introduced into the chamber, able to reduces with the same power density the deposition rate until to a value of about 90% of the deposition rate in pure Argon atmosphere; this flux of Ethylene is equivalent to a partial pressure of about 6 x 10⁻⁵ Torr;
- then the deposition is performed maintaining these parameters constant until a thickness of about 1,5 micron;
- then, both the DC and RF supply and the flux of the process gases are interrupted; and after, the vent of the chamber the substrates are demounted from the substrates holder and are so completed.

2. Process, for coatings for osteointegration of surgical prosthesis, to perform into a Ion Plating Plasma Assisted plant, from a source "reactive Magnetron Sputtering", as for Claim 1, **characterized by** the fact that the composition of the deposited film is a mixture of Titanium carbide and Titanium dioxide, TiO₂, Titanium sub-oxides, TiOx, and in addition Carbon bonded with other Carbon atoms and/or Oxygen atoms.

3. Process, for coatings for osteointegration of surgical prosthesis, as for Claim 1, **characterized by** the fact that in the chemical bonds of the main elements which constitute the treatment, the typical percentages of the Titanium compounds are : TiC between 35% and 38%, TiO₂ between 30% and 37%, TiOx between 26% and 34%, and relatively to the Carbon compounds there is a prevalence of the bond C-C around 50%, with values around 35% of bonds with Titanium and the remaining percentage with Oxygen.

4. Process, for coatings for osteointegration of surgical prosthesis, as for Claim 1, **characterized by** the fact that the Magnetron Sputtering source (2) is supplied in direct voltage, while the biased substrate holder (5) is biased through a RF supplier, typically at 13,56 MHz.

5. Process, for coatings for osteointegration of surgical prosthesis, as for Claim 1, **characterized by** the fact that the biased substrate holder (5) can be fixed or rotating in front of the source (2), preferably at a distance between five and fifteen centimetres.

6. Process, for coatings for osteointegration of surgical prosthesis, as for the preceding claims, **characterized by** the fact that the material on the Magnetron Sputtering source (2) is typically Titanium; the process atmosphere is constituted, in some phases, by Argon and Ethylene and the prosthesis to treat, typically in Titanium, are roughened through a sandblasting process using micro-spheres, typically in Zirconium Dioxide, ZrO₂, with a optimal diameter of about 120 microns.

7. Process, for coatings for osteointegration of surgical prosthesis, as for Claim 4. **characterized by** the fact that both the magnetron source and the biased substrate holder (5) are powered by a DC voltage or by any kind of alternate or pulsed frequency.

8. Process, for coatings for osteointegration of surgical prosthesis, as for Claim 1, **characterized by** the fact that the sputtering source can be also different from a magnetron.
